# EUROPEAN PATENT APPLICATION

(11) **EP 3 549 620 A1**
(43) Date of publication of application: **09.10.2019**
(21) Application number: 18165615.8
(22) Date of filing: 04.04.2018
(51) Int. Cl.: A61L 31/02, A61L 31/08, A61L 31/10, A61L 31/14

(54) **COATED IMPLANTABLE MEDICAL DEVICE AND COATING METHOD**

(71) Applicant: BIOTRONIK SE & Co. KG, 12359 Berlin (DE)
(72) Inventor: Frotscher, Matthias, 18109 Rostock (DE); Bosselmann, Marco, 18059 Rostock (DE); Momma, Carsten, 18057 Rostock (DE); von Arx, Jeffrey A., Lake Oswego, OR Oregon 97035 (US); Suwito, Wantjinarjo, West Linn, OR Oregon 97068 (US); Muessig, Dirk, West Linn, OR Oregon 97068 (US); Kraetschmer, Hannes, West Linn, OR Oregon 97068 (US)
(74) Representative: Keck, Hans-Georg

(57) **Abstract**

An implantable medical device (1) which is at least partially coated with a metal-ion release inhibiting coating material is disclosed. Also, a method for coating an implantable medical device is disclosed.

## Description

The disclosure relates to a coated implantable medical device and coating method.

### Background

When a medical device is implanted in a patient, biocompatibility is always an issue. In particular, metal ions released from the device into body tissue of the patient may cause an inflammatory reaction which may interfere with the healing process.

Document US 2013/0253345 A1 discloses an implantable medical device with a set of fixation tines. The tines are made of nitinol and are coated with a radiopaque coating, a tissue growth promoter or a tissue growth inhibitor.

Document US 5,238,866 A relates to an amorphous semi-conductive surface coating for a cardiovascular implant in order to reduce the risk of valve occlusions by thrombus, thromboembolism and anti-coagulant-related hemorrhage.

Document EP 0 371 908 A1 discloses an implant with a semiconducting coating, for example amorphous silicon carbide, in order to form an antithrombotic surface.

Document US 2010/0114284 A1 discloses an electrode line which is partially coated with an antithrombotic silicon carbide coating.

### Summary

It is an object to provide improved technologies for implantable devices. In particular, tolerability of the implantable device shall be improved.

An implantable medical device according to claim 1 and a method according to claim 12 are provided. Further embodiments are subject matter of dependent claims.

In one aspect, an implantable medical device which is at least partially coated with a metal-ion release inhibiting coating material is provided.

In another aspect, a method for coating an implantable medical device is disclosed. The method comprises steps of providing the implantable medical device, and depositing a metal-ion release inhibiting coating material on at least a portion of the device.

The implantable medical device may comprise a housing. The housing may comprise a metallic material. The housing may be at least partially coated with the metal-ion release inhibiting coating material.

The implantable medical device may comprise a fixation element for fixing the implantable medical device to body tissue. The fixation element may comprise a metallic material. The fixation element may be at least partially coated with the metal-ion release inhibiting coating material. The fixation element may be arranged on the housing.

The method may further comprise steps of: providing a fixation element for fixing the implantable medical device to body tissue, wherein the fixation element comprises a metallic material, and depositing a metal-ion release inhibiting coating material on at least a portion of the fixation element.

A metal-ion release inhibiting coating material is a material which suppresses a release of metal ions from the fixation element. Hereby, a transfer of metal ions from the medical device (e.g. from the fixation element) to body tissue is reduced or completely inhibited.

The fixation element may be made of a metallic material. The fixation element may be completely coated with the coating material.

In one embodiment, the fixation element may comprise a shape memory alloy, e.g. nitinol. The fixation element may be made of a shape memory alloy, in particular nitinol. Nickel titanium, also known as nitinol, is a metal alloy of nickel and titanium, where the two elements are present in roughly equal atomic percentage.

The coating material may be a Ni-ion release inhibiting material, thus, a material which suppresses release of Ni ions (e.g. from the fixation element).

The coating material may be an amorphous coating material.

The coating material may be selected from the group of: a-SiₓC₁₋ₓ:H (amorphous hydrogenated silicon carbide, in the following called "a-SiC coating"), TiN (titanium nitride), diamond-like carbon, and parylene.

The fixation element may be formed by one or more tines. Alternatively, the fixation element may be provided as a screw element.

The coating material may have a thickness of 1 - 1000 nm, e.g. 80 - 200 nm.

The implantable medical device may be an intracardiac pacing system. The intracardiac pacing system is implanted in the heart, e.g. in the ventricle or in the atrium. Thus, the fixation element anchors the intracardiac pacing system in heart tissue, e.g. the myocardium.

The intracardiac pacing system may comprise a lead extension with a further fixation element for fixing the lead extension to body tissue, wherein the further fixation element may comprise a metallic material, and wherein the further fixation element may be at least partially coated with the metal-ion release inhibiting coating material. For the further fixation element and for the coating material of the further fixation element, the embodiments relating to the fixation element and to the coating of the fixation element disclosed herein are also applicable.

In one embodiment, the implantable medical device is an intracardiac pacing system which comprises several tines (also called a tine array) which may protrude from a cap of a header of the intracardiac pacing system. The tines comprise nitinol (or are made of nitinol) and are partially or completely coated with a-SiₓC₁₋ₓ:H. The a-SiC coating reduces the amount of released Ni ions and leads to an improvement of the overall biocompatibility of the intracardiac pacing system.

The features disclosed herein in regard with the device may also apply to the method and vice versa.

### Description of embodiments

Following, exemplary embodiments are described with reference to figures. Here show:
- Fig. 1: a first embodiment of an intracardiac pacing system, and
- Fig. 2: a second embodiment of an intracardiac pacing system.

Same reference numerals are used for same components.

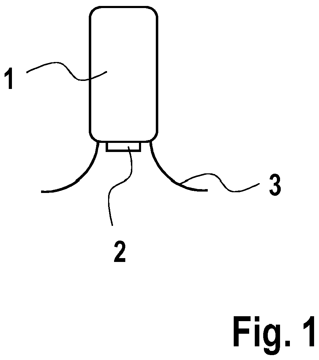

Fig. 1 shows an intracardiac pacing system 1 with a pacing electrode 2 and tines 3. The pacing electrode 2 is disposed on a lower end of the intracardiac pacing system. The tines 3 protrude from the lower end and form a tine array. After implantation of the intracardiac pacing system 1, the system is fixed in the heart (e.g. in the ventricle or in the atrium) by the tines 3 which penetrate heart tissue (e.g. the myocardium).

The tines 3 are used to anchor the intracardiac pacing system in the myocardium and ensure contact of the pacing electrode 2 with the tissue. The tines 3 are in permanent contact with tissue following implantation. The tines 3 are also in close proximity to the pacing electrode 2. Making the tines more biocompatible leads to less body rejection. This may result in a slower endo encapsulation rate, and / or a lower pacing threshold.

The tines 3 are made of nitinol and at least partially coated with a-SiₓC₁₋ₓ:H. In one embodiment, the tines 3 are completely coated with the a-SiC coating. Nitinol is known to elute Ni ions, which may cause harm in the human body. By applying the a-SiC coating on the surface of the tines 3, the amount of released Ni into the tissue and corresponding inflammatory reactions can be significantly reduced. By covering the surface of the tines 3 with the a-SiC coating, the immune system does not react as much to the intracardiac pacing system 1 (being a foreign object in the body) as it may do without the coating. The a-SiC coating has a thickness of 80 - 200 nm. The nitinol matrix of the base metal will be no longer in contact with body fluids and tissue.

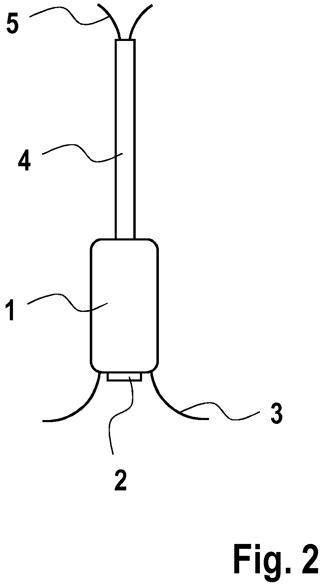

In the embodiment shown in Fig. 2, the intracardiac pacing system 1 comprises a lead extension 4. At a distal end of the lead extension 4, further tines 5 are disposed. The further tines 5 are also made of nitinol and (partially or completely) coated with an a-SiC coating. In this embodiment, the main body of the intracardiac pacing system 1 may be anchored in the ventricle by the tines 3 and the lead extension may be anchored in the atrium by the further tines 5. The intracardiac pacing system 1 may be used in a VDD (ventricular pacing, dual chamber sensing, triggered and inhibited mode) and / or DDD (dual chamber pacing and sensing, triggered and inhibited mode) pacing embodiment.

Following, a procedure for coating the tines 3 and/or the further tines 5 with an a-SiC coating is described. The surface coating of the tines/further tines is produced by way of a PECVD (plasma enhanced chemical vapor deposition) process. As coating material, amorphous hydrogenated silicon carbide (a-SiₓC₁₋ₓ:H) is used. A suitable a-SiC coating can be obtained, if the supplied process gases of the PECVD process are 10% silane (SiH₄) diluted with hydrogen as the silicon supply, and pure methane (CH₄) for carbon supply alloying together with 0.1% of phosphine (PH₃) also diluted with hydrogen. Suitable process parameters are:

| | |
|---|---|
| - pressure range: | 0.02 - 1 mbar; |
| - gas composition: | 50 - 80% methane, 100 - 0% silane, 0 - 2% phosphine; |
| - gas flow: | 10 - 50 sccm silane; |
| - substrate temperature: | 0 - 350°C. |

The features disclosed in the specification, the claims and the figures may be relevant for realizing embodiments either alone or in any combination with each other.

### List of reference numerals:

- 1: intracardiac pacing system
- 2: pacing electrode
- 3: tines
- 4: lead extension
- 5: further tines

## Claims

1. An implantable medical device (1) which is at least partially coated with a metal-ion release inhibiting coating material.

2. The device of claim 1, further comprising a fixation element (3) for fixing the implantable medical device (1) to body tissue, wherein the fixation element (3) comprises a metallic material, and wherein the fixation element (3) is at least partially coated with the metal-ion release inhibiting coating material.

3. The device of claim 2, wherein the fixation element (3) comprises a shape memory alloy.

4. The device of claim 3, wherein the fixation element (3) comprises nitinol.

5. The device of one of the preceding claims, wherein the coating material is a Ni-ion release inhibiting material.

6. The device of one of the preceding claims, wherein the coating material is an amorphous coating material.

7. The device of one of the preceding claims, wherein the coating material is selected from the group of: a-SiₓC₁₋ₓ:H, TiN, diamond-like carbon, and parylene.

8. The device of one of the claims 2 to 7, wherein the fixation element (3) is formed by one or more tines.

9. The device of one of the preceding claims, wherein the implantable medical device is an intracardiac pacing system.

10. The device of claim 9, wherein the intracardiac pacing system (1) comprises a lead extension (4) with a further fixation element (5) for fixing the lead extension (4) to body tissue, wherein the further fixation element (5) comprises a metallic material, and wherein the further fixation element (5) is at least partially coated with the metal-ion release inhibiting coating material.

11. The device of one of the preceding claims, wherein the coating material has a thickness of 1 to 1000 nm.

12. A method for coating an implantable medical device, comprising steps of:
- providing the implantable medical device (1), and
- depositing a metal-ion release inhibiting coating material on at least a portion of the device (1).

13. The method of claim 12, further comprising steps of:
- providing a fixation element (3) for fixing the implantable medical device (1) to body tissue, wherein the fixation element (3) comprises a metallic material, and
- depositing the metal-ion release inhibiting coating material on at least a portion of the fixation element (3).
